# FASCICULE DE BREVET EUROPEEN

(11) **EP 0 577 506 B1**
(45) Date de publication et mention de la délivrance du brevet: **30.07.1997**
(21) Numéro de dépôt: 93401685.8
(22) Date de dépôt: 30.06.1993
(51) Int. Cl.: A61K 7/48, A61K 7/06, C11D 3/32, C11D 1/52

(54) **Utilisation dans des solutions d'agents tensioactifs de carbamates d'aminopolyols en tant qu'épaississants**
Verwendung von Aminopolyol-carbamaten als Verdickungsmittel in Tensidlösungen
Use of aminopolyol carbamates as thickeners in solutions of surface active agents

(30) Priorité: 02.07.1992 FR 9208176
(43) Date de publication de la demande: 05.01.1994
(73) Titulaire: L'OREAL, 75008 Paris (FR)
(72) Inventeur: Vanlerberghe, Guy, F-77410 Claye-Souilly (FR); Mahieu, Claude, F-75017 Paris (FR); Morancais, Jean-Luc, F-77330 Ozoir la Ferriere (FR)
(74) Mandataire: Casalonga, Axel

(56) Documents cités:
- EP-A- 0 242 918
- EP-A- 0 242 919
- WO-A-92/05764
- BE-A- 543 132
- FR-A- 2 379 283
- US-A- 2 040 997

## Description

L'invention est relative à l'utilisation dans des solutions d'agents tensioactifs de carbamates d'aminopolyols en tant qu'épaississants, ainsi qu'à des compositions de lavage et de nettoyage contenant des agents tensioactifs et ces composés.

La structure des carbamates d'aminopolyols est déjà connue et décrite dans le brevet US 2 040 997.

On recherche dans le domaine de l'industrie cosmétique des produits de lavage présentant des consistances suffisamment épaisses de sorte que le composé détergent reste en contact avec le corps ou les cheveux sans couler dès l'application, afin d'éviter sa perte et, dans le cas des shampooings ou des démaquillants, le contact accidentel avec les yeux. Toutefois la consistance du produit de lavage doit aussi permettre un écoulement aisé à partir du flacon dans lequel il est contenu.

Un bon produit de lavage doit donc avoir une viscosité telle que l'écoulement soit commode à partir du flacon, mais suffisamment lent sur les cheveux ou le corps pour éviter les inconvénients précités.

Des solutions ont déjà été proposées pour résoudre ce problème, telles l'ajout de composés amphiphiles de structures particulières; il s'agit de dérivés d'acides gras, soit estérifiés par des chaînes polyoxyéthylénées, soit amidifiés par des amines polyhydroxylées. Un des composés de cette classe, le plus fréquemment utilisé est le mélange d'acides gras constitutifs de l'acide de coprah amidifié par la diéthanolamide. Plus récemment, il a été découvert, dans la demande de brevet EP 285 768, que le remplacement de la diéthanolamide par un reste aminopolyhydroxylé dérivant d'un ose ou polyose, et plus précisémment la N-méthyl D-glucamine dérivant du glucose améliorait l'épaississement de solution de tensio-actifs.

Les propriétés idéales recherchées pour cette classe de produits étant d'obtenir un épaississement maximum du produit de lavage pour une quantité réduite d'additif. Jusqu'à présent une quantité importante d'électrolyte, tel que le chlorure de sodium, associé aux composés dérivés d'acides gras afin d'exhalter leur activité, était indispensable pour obtenir des épaississements satisfaisants, mais néanmoins limités.

Le but de la présente invention est de fournir des compositions contenant des agents tensioactifs suffisamment épaisses sans toutefois présenter les inconvénients précités.

Il a été découvert de façon surprenante, ce qui fait l'objet de la présente invention, que le remplacement de la fonction amide dérivant des acides gras décrits ci-dessus, par une fonction carbamate, dérivant des alcools gras, permettait, de façon surprenante, d'améliorer l'épaississement de solutions de tensioactifs. Les solutions d'agents tensioactifs contenant les carbamates conformes à l'invention présentent une consistance suffisamment épaisse même avec une quantité minimale d'additif, notamment un électrolyte, tout en permettant un écoulement commode du produit.

La présente invention a donc pour objet l'utilisation, dans des solutions d'agents tensioactifs, de carbamates d'aminopolyols en tant qu'épaississants.

Un autre objet de l'invention consiste en une composition de lavage et/ou de nettoyage contenant des agents tensioactifs et, à titre d'agent épaississant, au moins un carbamate d'aminopolyol.

D'autres objets de l'invention apparaîtront à la lecture de la description et des exemples qui suivent.

Les composés utilisés à titre d'agents épaississants dans des solutions d'agents tensioactifs sont des carbamates d'aminopolyols de formule générale : 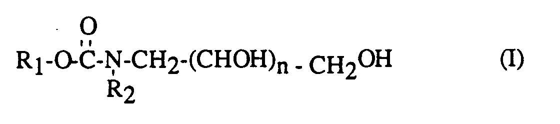 dans laquelle :
R₁ représente un radical alkyle linéaire saturé en C₈₋C₁₃;
R₂ représente un hydrogène ou un radical alkyle en C₁-C₁₀;
n est un entier compris entre 1 et 5.

Parmi les significations préférées des substituants définis ci-dessus, R₂ représente un radical alkyle en C₁-C₆ et n vaut 4 ce qui correspond pour le radical 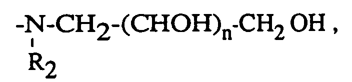 aux aminopolyols eux-même dérivés d'un ose naturel tel que le glucose, le galactose, le mannose..., et plus communément appelés glucamine, galactamine, mannosamine...

Le choix de R₁, R₂ et n pour les composés carbamates d'aminopolyols, est fait en fonction de leur solubilité dans les solutions tensioactives concernées.

Les carbamates d'aminopolyols de formule générale (I) plus particulièrement préférés sont les carbamates de N-méthyl glucamine, parmi lesquels on peut citer :
-la N-décyloxycarbonyl N-méthyl glucamine.

Les carbamates d'aminopolyols de formule générale (I) peuvent être obtenus de plusieurs manières :

Ils peuvent être préparés en traitant l'aminopolyol en question, obtenu par la réduction en présence d'ammoniaque ou d'amines primaires de l'ose naturel correspondant, par du chloroformiate d'alkyle linéaire saturé en C₈-C₁₃.

Un autre procédé de préparation de ces carbamates utilisables selon l'invention consiste à faire réagir un uréthane cyclique d'un acide N-alkyl N-sorbityl carbamique avec un alcanol. Ce prodédé est décrit dans le brevet USP 2 408 402.

Les agents tensioactifs utilisés dans des solutions épaissies conformément à l'invention peuvent être anioniques, non-ioniques, ou des mélanges d'agents tensioactifs anioniques et non-ioniques.

Les agents tensio-actifs anioniques utilisables selon l'invention sont choisis parmi :

Les sels alcalins, les sels d'ammonium, les sels d'amines, les sels d'aminoalcools, les sels de magnésium des composés suivants :
- les alkylsulfates, alkyléther sulfates, alkylamidoéthersulfates, alkylarylpolyéthersulfates, monoglycérides sulfates;
- les alkylsulfonates, alkylamides sulfonates, alkylarylsulfonates, oléfines sulfonates, paraffines sulfonates;
- les alkylsulfosuccinates, les alkyléthersulfosuccinates, les alkylamides sulfosuccinates:
- les alkylsulfosuccinamates;
- les alkylsulfoacétates;
- les alkylphosphates, alkyléther phosphates;
- les acylsarcosinates, les acyliséthionates, N-acyltaurates.

Le radical alkyle ou acyle de ces différents composés est généralement constitué par une chaîne carbonée comportant de 12 à 20 atomes de carbone.

On peut également citer : des sels d'acide gras tels que des acides oléique, ricinoléïque, palmitique, stéarique; les acides d'huile de coprah ou d'huile de coprah hydrogénée; les acyl lactylates dont le radical acyle comporte 8 à 20 atomes de carbone.

Parmi les agents tensio-actifs non-ioniques on peut citer plus particulièrement : des alcools, des alkylphénols et des acides gras polyéthoxylés, polyoxypropylénés ou polyglycérolés à chaîne grasse comportant 8 à 18 atomes de carbone. Le nombre de groupements oxyde d'éthylène, oxyde de propylène étant compris entre 2 et 50 et le nombre de groupements glycérol étant compris entre 2 et 30.

On peut également citer des copolymères d'oxydes d'éthyléne et de propylène, des condensats d'oxydes d'éthylène et de propylène sur des alcools gras, des amides gras polyéthoxylés (de préférence à 2 a 30 moles d'oxyde d'éthylène), des amines grasses polyéthoxylées (de préférence de 2 à 30 moles d'oxyde d'éthylène), des éthanolamides, des esters d'acides gras du sorbitan oxyéthylénés (de préférence 2 à 30 moles d'oxyde d'éthylène) ou non, des esters d'acides gras du polyéthylène glycol, des triesters phosphoriques, des esters d'acides gras de sucrose, les alkylpolyglycosides, des oxydes d'amines grasses.

L'activité épaississante des carbamates d'aminopolyols utilisés conformément à l'invention est déterminée par des mesures de viscosité à différentes vitesses de cisaillement à l'aide d'un rhéomètre fonctionnant à vitesse de cisaillement imposée.

Par ces mesures il a été constaté que l'activité épaississante des carbamates d'aminopolyols de formule (I) définie ci-dessus, est supérieure à celles des épaississants de solution d'agents tensioactifs de l'art antérieur.

La présente invention a également pour objet des compositions de lavage et/ou de nettoyage contenant dans un milieu aqueux des agents tensioactifs et, à titre d'agent épaississant, au moins un carbamate d'aminopolyol de formule générale (I) définie ci-dessus.

Les composés carbamates d'aminopolyols de formule générale (I) définie, ci-dessus, sont présents dans ces compositions, dans des concentrations variant de 0,1 à 40 % en poids par rapport au poids total de la composition, et de préférence 0,1 à 10 % en poids.

Les compositions conformes à l'invention contiennent également en association avec les composés carbamates d'aminopolyols de formule générale (I) définie ci-dessus, des électrolytes qui permettent d'exhalter leur activité en tant qu'épaississants. Parmi ces électrolytes on peut citer le chlorure de sodium ou le sulfate de sodium.

Les électrolytes utilisés dans les compositions conformes à l'invention sont présents de préférence dans des concentrations allant de 0,01 à 10 % en poids par rapport au poids total de la composition.

Les agents tensioactifs sont de préférence des agents tensioactifs anioniques et/ou non ioniques tels que définis ci-dessus.

Ces agents tensioactifs sont présents dans les compositions de la présente invention dans des proportions comprises entre 1 et 80 %, de préférence entre 1 et 50 % en poids par rapport au poids total de la composition.

Le rapport en poids de carbamates d'aminopolyols de formule (I) définie ci-dessus, aux agents tensioactifs utilisés dans les compositions conformes à l'invention, est compris entre 0,05 et 1.

Le milieux aqueux peut être constitué par de l'eau ou un mélange d'eau et de solvants organiques pour solubiliser les composants qui ne seraient pas suffisamment solubles dans l'eau. Parmi ces solvants, on peut citer à titre d'exemple, les alcanols inférieures en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

D'autres agents épaississants différents des carbamates d'aminopolyols de formule (I) peuvent être aj outés dans les compositions conformes à l'invention, tels que ceux choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces compositions peuvent également contenir d'autres adjuvants cosmétiquement acceptables, tels que par exemple des agents séquestrants, des parfums, des tampons, des agents de traitement, des agents de conditionnement, des agents conservateurs, etc.

Les compositions conformes à l'invention peuvent se présenter sous des formes diverses, telles que sous forme de liquide, de crème, de gel ou sous toute autre forme appropriée, et peuvent être conditionnées dans des flacons aérosols et en présence d'un agent propulseur pour former des mousses.

Le pH des compositions conformes à l'invention varie entre 3 et 8. Il est ajusté à la valeur à l'aide d'agents alcalinisants et d'agents acidifiants habituellement utilisés en cosmétique.

La composition de l'invention est utilisée pour le lavage ou le nettoyage des matières kératiniques plus particulièrement des matières kératiniques humaines, notamment les cheveux et la peau, après quoi on rince ces dernières et on les sèche.

Les exemples qui suivent sont destinés à illustrer l'invention sans pour autant présenter un caractère limitatif.

### Exemple 1 :

### Préparation du 1-[méthyl [(décyloxy) carbonyl]amino]-deoxy-D-glucitol (ou N-décyloxycarbonyl-N-méthyl glucamine)

Dans un réacteur, on dissout 23,4 g de N-méthyl glucamine (0,12 mole) dans un mélange de 60 ml d'eau et de 80 ml de tétrahydrofurane, puis on ajoute et disperse 40 g d'hydrogénocarbonate de sodium (0,48 mole).

En maintenant la température du mélange réactionnel à 20°C, on ajoute goutte à goutte 26,4 g de chloroformiate de décyle (commercialisé par la SNPE) (0,12 mole), puis on laisse pendant 3 heures sous agitation à température ambiante et une nuit au repos.

Le mélange réactionnel est alors filtré (le produit insoluble est essentiellement de la N-méthyl glucamine) et le filtrat est refroidi à O°C pendant une nuit. Le produit solide récupéré est recristallisé dans 300 ml d'acétone (un insoluble est éliminé par filtration à chaud).

Après refroidissement à O°C pendant 12 heures, le produit cristallisé est récupéré et séché. 24 g de produit pur est obtenu.

Fusion: 59,8°C

| Analyse élémentaire: | | | | |
|---|---|---|---|---|
| | C | H | N | O |
| Théorie | 56,97 | 9,83 | 3,69 | 29,51 |
| Trouvé | 56, 88 | 9,91 | 3,50 | 29,48 |

### EXEMPLES DE COMPOSITIONS

### EXEMPLE 1: Shampooing

| | |
|---|---|
| - Alkylsulfate de triéthanolamine (C₁₂/C₁₄ 70/30) vendu sous la dénomination "EMPICOL TL 40/FL" par la Société MARCHON | 30,0 g |
| - Composé de l'exemple 1 | 1,33 g |
| - Chlorure de sodium | 1,0 g |

| - Conversateurs, parfum | |
|---|---|
| - Eau q.s.p. | 100,0 g |
| - Triéthanolamine qs pH 7 | |

On obtient un shampooing épaissi limpide

### EXEMPLE 2 : Gel douche

| | |
|---|---|
| - Alkyl éther sulfate de sodium (C₁₂/C₁₄ 70/30) vendu sous la dénomination "EMPICOL" ESB/3FL" par la Société MARCHON | 80,0 g |
| - Composé de l'exemple 1 | 4,0 g |
| - Chlorure de sodium | 3,0 g |

| - Conversateurs, parfum | |
|---|---|
| - Eau q.s.p. | 100,0 g |
| - Acide chlorhydrique qs pH 7 | |

On obtient un gel douche limpide.

### EXEMPLE 3 : Shampooing

| | |
|---|---|
| - Alkyl(C₉/C₁₀/C₁₁)-20/40/40)-polyglucoside (1,4) vendu sous la dénomination APG 300 à 50 % de MA par la société HENKEL | 10 g MA |
| - Composé de l'exemple 1 | 7 g |
| - Chlorure de sodium | 5 g |

| - Conversateurs, parfum qs | |
|---|---|
| - Eau q.s.p. | 100 g |

## Revendications

1. Utilisation, en tant qu'agent épaississant dans des solutions d'agents tensioactifs, de carbamates d'aminopolyols répondant à la formule générale : 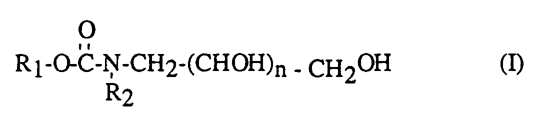 dans laquelle :
R₁ représente un radical alkyle linéaire saturé en C₈-C₁₃;
R₂ représente un atome d'hydrogène ou un radical alkyle en C₁-C₁₀;
n est un nombre entier compris entre 1 et 5.

2. Utilisation selon la revendication 1, caractérisée en ce que dans la formule (I) définie ci-dessus, R₂ désigne un radical alkyle en C₁-C₆ et n est égal à 4.

3. Utilisation selon la revendication 1 ou 2, caractérisée en ce que les carbamates d'aminopolyols sont des carbamates de N-méthyl-glucamine.

4. Utilisation selon l'une quelconque des revendications 1 à 3, caractérisé en ce que le carbamate utilisé est la N-décyloxycarbonyl-N-méthyl glucamine.

5. Utilisation selon l'une quelconque des revendications 1 à 4, caractérisée en ce que les agents tensioactifs sont des agents tensioactifs anioniques et/ou des agents tensioactifs non ioniques.

6. Utilisation selon la revendication 5, caractérisée en ce que les agents tensioactifs anioniques sont choisis parmi les sels alcalins, les sels d'ammonium, les sels d'amines, les sels d'aminoalcools, les sels de magnésium des composés suivants :
- les alkylsulfates, alkyléther sulfates, alkyl amidoéthersulfates, alkylarylpolyéthersulfates , monoglycérides sulfates;
- les alkylsulfonates, alkylamides sulfonates, alkylarylsulfonates, oléfines sulfonates, paraffines sulfonates;
- les alkylsulfosuccinates, les alkyléthersulfosuccinates, les alkylamides sulfosuccinates;
- les alkylsulfosuccinamates ;
- les alkylsulfoacétates;
- les alkylphosphates, alkyléther phosphates;
- les acylsarcosinates, les acyliséthionates, N-acyltaurates;
le radical alkyle ou acyle étant en C₁₂-C₂₀;
les sels d'acides gras; les acides d'huile de coprah ou d'huile de coprah hydrogénée; les acyl lactylates dont le radical acyle est en C₈-C₂₀;

7. Utilisation selon la revendication 5 ou 6, caractérisée en ce que les agents tensioactifs non ioniques sont les alcools, les alkylphénols et les acides gras polyéthoxylés, polyoxypropylénés ou polyglycérolés à chaîne grasse comportant 8 à 18 atomes de carbone; des copolymères d'oxydes d'éthylène et de propylène, des condensats d'oxydes d'éthylène et de propylène sur des alcools gras, des amides gras polyéthoxylés, des amines grasses polyéthoxylées, des éthanolamides, des esters d'acides gras du sorbitan oxyéthylénés ou non, des esters d'acides gras du polyéthylène glycol, des triesters phosphoriques, des esters d'acides gras de sucrose, les alkylpolyglycosides, des oxydes d'amines grasses.

8. Composition de lavage ou de nettoyage contenant dans un milieu aqueux des agents tensioactifs et au moins, un carbamate d'aminopolyol de formule générale : 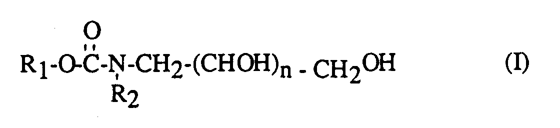 dans laquelle :
R₁ représente un radical alkyle linéaire saturé en C₈-C₁₃;
R₂ représente un atome d'hydrogène ou un radical alkyle en C₁-C₁₀;
n est un nombre entier compris entre 1 et 5.

9. Composition selon la revendication 8, caractérisée en ce qu'elle contient des agents tensioactifs anioniques et/ou non ioniques tels que définis dans les revendications 6 et 7.

10. Composition selon la revendication 8 ou 9, caractérisée en ce que le carbamate d'aminopolyol est présent dans des concentrations variant de 0,1 à 40 % en poids par rapport au poids total de la composition.

11. Composition selon l'une quelconque des revendications 8 à 10, caractérisée en ce que les agents tensiocatifs sont présents en des concentrations allant de 1 à 80 % en poids par rapport au poids total de la composition.

12. Composition selon la revendication 11, caractérisée en ce que les agents tensioactifs sont présents en des concentrations allant de 1 à 50 % en poids par rapport au poids total de la composition.

13. Composition selon l'une quelconque des revendications 8 à 12, caractérisée en ce que le rapport en poids de carbamate aux agents tensioactifs est compris entre 0,05 et 1.

14. Composition selon l'une quelconque des revendications 8 à 13, caractérisée en ce qu'elle contient des électrolytes dans des concentrations allant de 0,01 à 10 % en poids par rapport au poids total de la composition.

15. Composition selon l'une quelconque des revendications 8 à 14, caractérisée en ce qu'elle contient des solvants organiques, des agents épaississants différents des carbamates d'aminopolyols de formule (I), des agents séquestrants, des parfums, des tampons, des agents de traitement, des agents de conditionnement, des agents conservateurs et tout adjuvant cosmétiquement acceptable.

16. Composition selon l'une quelconque des revendications 8 à 15, caractérisée en ce qu'elle se présente sous forme de liquide, de gel, de crème ou sous tout autre forme appropriée et qu'elle peut être conditionnée en flacon aérosol et en présence d'un agent propulseur pour former des mousses.

17. Composition selon l'une quelconque des revendications 8 à 16, caractérisée en ce que sa valeur de pH est comprise entre 3 et 8.

18. Procédé de lavage ou de nettoyage des matières kératiniques caractérisé par le fait que l'on applique sur ces matières au moins une composition telle que définie dans l'une quelconque des revendications 8 à 17, et qu'après un temps de pose on rince.

## Claims

1. Use, as thickening agent in surface-active agent solutions, of aminopolyol carbamates corresponding to the general formula: 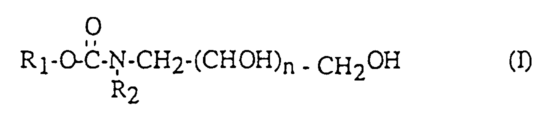 in which:
R₁ represents a linear, saturated C₈-C₁₃ alkyl radical;
R₂ represents a hydrogen atom or a C₁-C₁₀ alkyl radical;
n is an integer between 1 and 5.

2. Use according to Claim 1, characterized in that, in the formula (I) defined above, R₂ denotes a C₁-C₆ alkyl radical and n is equal to 4.

3. Use according to Claim 1 or 2, characterized in that the aminopolyol carbamates are carbamates of N-methylglucamine.

4. Use according to any one of Claims 1 to 3, characterized in that the carbamate used is N-decyloxycarbonyl-N-methylglucamine.

5. Use according to any one of Claims 1 to 4, characterized in that the surface-active agents are anionic surface-active agents and/or nonionic surface-active agents.

6. Use according to Claim 5, characterized in that the anionic surface-active agents are chosen from alkali metal salts, ammonium salts, amine salts, amino-alcohol salts or magnesium salts of the following compounds:
- alkyl sulfates, alkyl ether sulfates, alkylamido ether sulfates, alkyl aryl polyether sulfates, or monoglyceride sulfates;
- alkyl sulfonates, alkylamide sulfonates, alkyl aryl sulfonates, olefin sulfonates, or paraffin sulfonates;
- alkyl sulfosuccinates, alkyl ether sulfosuccinates, or alkylamide sulfosuccinates;
- alkyl sulfosuccinamates;
- alkyl sulfoacetates;
- alkyl phosphates, or alkyl ether phosphates;
- acyl sarcosinates, acyl isethionates, or N-acyl taurates;
the alkyl or acyl radical being C₁₂-C₂₀;
fatty acid salts; coconut oil acid or hydrogenated coconut oil acid; acyl lactylates in which the acyl radical is C₈-C₂₀.

7. Use according to Claim 5 or 6, characterized in that the nonionic surface-active agents are polyethoxylated, polyoxypropylenated or polyglycerolated alcohols, alkylphenols and fatty acids containing fatty chains comprising 8 to 18 carbon atoms; copolymers of ethylene oxide and of propylene oxide, condensates of ethylene oxide and propylene oxide with fatty alcohols, polyethoxylated fatty amides, polyethoxylated fatty amines, ethanolamides, oxyethylenated or nonoxyethylenated fatty acid esters of sorbitan, fatty acid esters of polyethylene glycol, phosphoric triesters, fatty acid esters of sucrose, alkylpolyglycosides or fatty amine oxides.

8. Washing or cleaning composition containing, in an aqueous medium, surface-active agents and at least one aminopolyol carbamate of general formula: 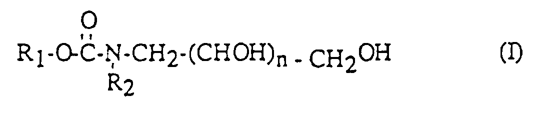 in which:
R₁ represents a linear, saturated C₈-C₁₃ alkyl radical;
R₂ represents a hydrogen atom or a C₁-C₁₀ alkyl radical;
n is an integer between 1 and 5.

9. Composition according to Claim 8, characterized in that it contains anionic and/or nonionic surface-active agents as defined in Claim 6 and 7.

10. Composition according to Claim 8 or 9, characterized in that the aminopolyol carbamate is present in concentrations varying from 0.1 to 40 % by weight with respect to the total weight of the composition.

11. Composition according to any one of Claims 8 to 10, characterized in that the surface-active agents are present in concentrations ranging from 1 to 80 % by weight with respect to the total weight of the composition.

12. Composition according to Claim 11, characterized in that the surface-active agents are present in concentrations ranging from 1 to 50 % by weight with respect to the total weight of the composition.

13. Composition according to any one of Claims 8 to 12, characterized in that the ratio by weight of carbamate to the surface-active agents is between 0.05 and 1.

14. Composition according to any one of Claims 8 to 13, characterized in that it contains electrolytes in concentrations ranging from 0.01 to 10 % by weight with respect to the total weight of the composition.

15. Composition according to any one of Claims 8 to 14, characterized in that it contains organic solvents, thickening agents other than the aminopolyol carbamates of formula (I), sequestering agents, fragrances, buffers, treatment agents, conditioning agents, preserving agents, and any cosmetically acceptable adjuvant.

16. Composition according to any one of Claims 8 to 15, characterized in that it is provided in the liquid, gel or cream form or any other suitable form and in that it can be packaged in an aerosol container and in the presence of a propellant in order to form foams.

17. Composition according to any one of Claims 8 to 16, characterized in that its pH value is between 3 and 8.

18. Process for washing or cleaning keratinous materials, characterized in that there is applied to these materials at least one composition as defined in any one of Claims 8 to 17, and in that, after an exposure time, rinsing is carried out.

## Patentansprüche

1. Verwendung von Aminopolyolcarbamaten der allgemeinen Formel: worin gilt: 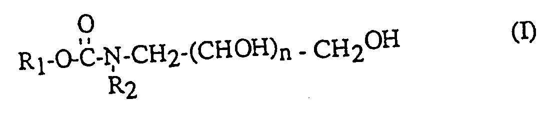
R₁ stellt einen linearen gesättigten C₈₋₁₃-Alkylrest dar;
R₂ stellt ein Wasserstoffatom oder einen C₁₋₁₀-Alkylrest dar;
n ist eine ganze Zahl von 1 bis 5,
als Verdickungsmittel in Lösungen oberflächenaktiver Mittel.

2. Verwendung gemäß Anspruch 1,
dadurch **gekennzeichnet**, daß
in der oben definierten Formel (I) R₂ einen C₁₋₆-Alkylrest bedeutet und n gleich 4 ist.

3. Verwendung gemäß Anspruch 1 oder 2,
dadurch **gekennzeichnet**, daß
die Aminopolyolcarbamate Carbamate von N-Methylglucamin sind.

4. Verwendung gemäß einem der Ansprüche 1 bis 3,
dadurch **gekennzeichnet**, daß
das eingesetzte Carbamat N-Decyloxycarbonyl-N-methylglucamin ist.

5. Verwendung gemäß einem der Ansprüche 1 bis 4,
dadurch **gekennzeichnet**, daß
die oberflächenaktiven Mittel anionische und/oder nicht-ionische oberflächenaktiven Mittel sind.

6. Verwendung gemäß Anspruch 5,
dadurch **gekennzeichnet**, daß
die anionischen oberflächenaktiven Mittel ausgewählt sind aus:
Alkali-, Ammonium-, Amin-, Aminoalkohol- und aus Magnesiumsalzen der folgenden Verbindungen:
- von Alkylsulfaten, Alkylethersulfaten, Alkylamidoethersulfaten, Alkylarylpolyethersulfaten und von Monoglyceridsulfaten;
- von Alkylsulfonaten, Alkylamidsulfonaten, Alkylarylsulfonaten, Olefinsulfonaten und von Paraffinsulfonaten;
- von Alkylsulfosuccinaten, Alkylethersulfosuccinaten und von Alkylamidsulfosuccinaten; sowie von
- Alkylsulfosuccinamaten;
- Alkylsulfoacetaten;
- Alkylphosphaten und Alkyletherphosphaten;
- Acylsarcosinaten, Acylisethionaten und von N-Acyltauraten, worin der Alkyl- oder Acylrest ein C₁₂₋₂₀-Rest ist;
aus Salzen von Fettsäuren; aus Säuren vom Öl von Kopra oder vom Öl von hydriertem Kopra, aus Acyllactylaten, deren Acylrest ein C₈₋₂₀-Rest ist.

7. Verwendung gemäß Anspruch 5 oder 6,
dadurch **gekennzeichnet**, daß
die nicht-ionischen oberflächenaktiven Mittel polyethoxylierte, polyoxpropylierte oder polyglycerierte Alkohole, Alkylphenole und Fettsäuren mit einer Fettkette von 8 bis 18 Kohlenstoffatomen, Copolymere aus Ethylen- und Propylenoxiden, Kondensate von Ethylen- und Propylenoxiden an Fettalkohole, polyethoxylierte Fettamide, polyethoxylierte Fettamine, Ethanolamide, gegebenenfalls oxethylierte Sorbitanfettsäureester, Fettsäureester von Polyethylenglycol, Phosphorsäuretriester, Fettsäureester von Sucrose, Alkylpolyglycoside und Oxide von Fettaminen sind.

8. Zusammensetzung zum Waschen oder Reinigen, enthaltend in einem wässrigen Medium oberflächenaktive Mittel und mindestens ein Aminopolyolcarbamat der allgemeinen Formel: 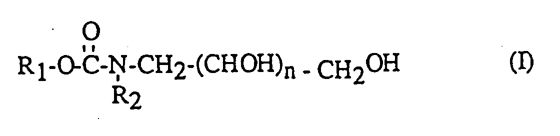 worin gilt:
R₁ stellt einen linearen gesättigten C₈₋₁₃-Alkylrest dar;
R₂ stellt ein Wasserstoffatom oder einen C₁₋₁₀-Alkylrest dar;
n ist eine ganze Zahl von 1 bis 5.

9. Zusammensetzung gemäß Anspruch 8,
dadurch **gekennzeichnet**, daß
sie die in den Ansprüchen 6 und 7 definierten anionischen und/oder nicht-ionischen oberflächenaktiven Mittel enthält.

10. Zusammensetzung gemäß Anspruch 8 oder 9,
dadurch **gekennzeichnet**, daß
das Aminopolyolcarbamat in Konzentrationen von 0,1 bis 40 Gew.% vorhanden ist, bezogen auf das Gesamtgewicht der Zusammensetzung.

11. Zusammensetzung gemäß einem der Ansprüche 8 bis 10,
dadurch **gekennzeichnet**, daß
die oberflächenaktiven Mittel in Konzentrationen von 1 bis 80 Gew.% vorhanden sind, bezogen auf das Gesamtgewicht der Zusammensetzung.

12. Zusammensetzung gemäß Anspruch 11,
dadurch **gekennzeichnet**, daß
die oberflächenaktiven Mittel in Konzentrationen von 1 bis 50 Gew.% vorhanden sind, bezogen auf das Gesamtgewicht der Zusammensetzung.

13. Zusamensetzung gemäß einem der Ansprüche 8 bis 12,
dadurch **gekennzeichnet**, daß
das Gewichtsverhältnis von Carbamat zu den oberflächenaktiven Mitteln 0,05 bis 1 beträgt.

14. Zusammensetzung gemäß einem der Ansprüche 8 bis 13,
dadurch **gekennzeichnet**, daß
sie Elektrolyte in Konzentrationen von 0,01 bis 10 Gew.% enthält, bezogen auf das Gesamtgewicht der Zusammensetzung.

15. Zusammensetzung gemäß einem der Ansprüche 8 bis 14,
dadurch **gekennzeichnet**, daß
sie organische Lösungsmittel, die sich von den Aminopolyolcarbamaten der Formel (I) unterscheiden, Sequestriermittel, Parfüm-Produkte, Puffermittel, Behandlungsmittel, Konditioniermittel, Konservierungsmittel sowie jeden weiteren kosmetisch geeigneten Hilfsstoff enthält.

16. Zusammensetzung gemäß einem der Ansprüche 8 bis 15,
dadurch **gekennzeichnet**, daß
sie in Form einer Flüssigkeit, eines Gels, einer Creme oder in jeder weiteren geeigneten Form vorliegt und auch in einem Aerosol-Fläschchen in Gegenwart eines Treibmittels zur Bildung von Schäumen zubereitet sein kann.

17. Zusammensetzung gemäß einem der Ansprüche 8 bis 16,
dadurch **gekennzeichnet**, daß
ihr pH-Wert 3 bis 8 beträgt.

18. Verfahren zum Waschen oder Reinigen keratinischer Materien,
dadurch **gekennzeichnet**, daß
man auf diese Materien mindestens eine in einem der Ansprüche 8 bis 17 definierte Zusammensetzung aufbringt und anwendet, worauf man nach einer Verweilzeit spült.
